# EUROPÄISCHE PATENTSCHRIFT

(11) **EP 3 437 579 B1**
(45) Veröffentlichungstag und Bekanntmachung des Hinweises auf die Patenterteilung: **12.07.2023**
(21) Anmeldenummer: 17184993.8
(22) Anmeldetag: 04.08.2017
(51) Int. Cl.: A61B 18/02, A61B 18/00

(54) **KRYOCHIRURGISCHES INSTRUMENT**
CRYOSURGICAL INSTRUMENT
INSTRUMENT CRYOCHIRURGICAL

(43) Veröffentlichungstag der Anmeldung: 06.02.2019
(60) Teilanmeldung: 23158476.4
(73) Patentinhaber: Erbe Elektromedizin GmbH, 72072 Tübingen (DE)
(72) Erfinder: Brodbeck, Achim, 72555 Metzingen (DE); Kronenthaler, Jörg, 72145 Hirrlingen (DE); Adler, Marcus, 72070 Tübingen (DE); Andel, Hanna, 72072 Tübingen (DE); Fischer, Klaus, 72202 Nagold (DE)
(74) Vertreter: Rüger Abel Patentanwälte PartGmbB

(56) Entgegenhaltungen:
- WO-A1-02/02026
- WO-A1-99/66970
- DE-A1-102008 024 946
- DE-T2- 69 906 320
- US-A- 5 759 182
- US-A1- 2013 310 822

## Beschreibung

Die Erfindung betrifft ein kryochirurgisches Instrument, das unter Ausnutzung des Joule-Thomson-Effekts arbeitet.

Aus dem Stand der Technik sind medizinische Instrumente bekannt, deren Arbeitsende abgekühlt wird, um damit physiologische oder therapeutische Effekte am Gewebe des Patienten zu erzeugen. Aus WO 02/02026 A1 beispielsweise ist eine Kryosonde bekannt, die eine Spitze zum Schneiden aufweist, wobei flüssiges Kältemittel an die Spitze geleitet wird, um diese abzukühlen. US 6 830 581 B2 beschreibt ein Wärmeübertragungselement zum Einführen in ein Blutgefäß, mit dem Blut in dem Gefäß abgekühlt werden soll, in dem der Spitze des Instruments abgekühltes Arbeitsmittel zugeführt wird.

Instrumente für die Kryochirurgie arbeiten beispielsweise durch das gezielte Einsetzen des Joule-Thomson-Effekts, wobei ein Fluid durch Drosselung eine Verminderung seiner Temperatur erfährt.

Aus DE 10 2008 024 946 A1 beispielsweise ist ein kryochirurgisches Instrument bekannt, das eine Zuführungsleitung zum Zuführen eines Fluids, insbesondere eines Gases, in eine Expansionskammer in dem Kopf der Sonde aufweist. An der Stirnseite der Zuführungsleitung ist eine Blende mit einer Öffnung angeordnet, durch die das Fluid aus der Zuführungsleitung in die Expansionskammer strömt und dabei entspannt wird, wobei sich das Fluid abkühlt. Damit wird die Sondenspitze abgekühlt. Das abgekühlte Fluid strömt von der Sondenspitze zurück durch eine Gasrückführungsleitung.

WO 2006/006986 A2 beschreibt ein kryochirugisches Instrument mit einer Röhre mit geschlossenem Ende. Innerhalb der Röhre ist eine Gaszuführungsleitung angeordnet, an deren Ende eine Kapillarröhre angeschlossen ist, deren Ende in eine Expansionskammer in der Spitze der Sonde mündet.

US 2012/0 130 359 A1 beschreibt ein Instrument zur Kryotherapie, mithilfe dessen Nerven am Einsatzort mit Kälte zu therapeutischen Zwecken beeinflusst werden können. Das Instrument weist einen Schaft auf, an dessen Ende ein Arbeitsabschnitt angeordnet ist. Durch den Schaft in den Arbeitsabschnitt erstreckt sich eine Zuführungsleitung zum Zuführen von Kühlmittel in den Arbeitsabschnitt. Am Ende der Zuführungsleitung kann eine Drosselblende oder eine Kapillarröhre angeordnet sein, mit der die Zuführungsleitung in eine Expansionskammer in dem Arbeitsbereich mündet.

US 2005/0016 188 A1 beschreibt ein Instrument zur kryochirurgischen Abtragung von Gewebe mit einem Kryokatheter mit einer Röhre, deren distales Ende verschlossen ist, wobei sich in der Röhre eine Zuführungsleitung zum Ende des Instruments erstreckt, wobei in dem Ende der Zuführungsleitung eine Kapillarröhre angeordnet ist, die in eine Kammer am distalen Ende des Instruments mündet. DE 699 06 320, WO 99/66970, US5759182 und US2013310822 offenbaren weiteren Stand der Technik.

Es ist Aufgabe der vorliegenden Erfindung, ein verbessertes kryochirurgisches Instrument anzugeben.

Diese Aufgabe wird mit einem kryochirurgischen Instrument nach Anspruch 1 gelöst, das beispielsweise zur Gewinnung einer Gewebeprobe eingerichtet sein kann. Das erfindungsgemäße kryochirurgische Instrument weist eine Zuführungsleitung zum Zuführen eines Arbeitsfluids, insbesondere eines Gases, in eine Expansionskammer auf, die vorzugsweise am distalen Ende des Instruments angeordnet ist. Die Zuführungsleitung weist einen Kapillarleitungsabschnitt auf, der in die Expansionskammer mündet. An die Expansionskammer ist eine Rückführungseinrichtung zum Rückführen von Gas aus der Expansionskammer angeschlossen. Die Zuführungsleitung weist wenigstens einen ersten Abschnitt und einen zweiten Abschnitt auf, die Leitungsabschnitte mit unterschiedlich großen Innenquerschnitten (Innenquerschnittsflächen) bilden. Die Innenquerschnitte legen den Strömungsquerschnitt für das Fluid durch die Zuführungsleitung in dem ersten und dem zweiten Abschnitt fest. Die Zuführungsleitung des erfindungsgemäßen Instruments ist derart gestaltet, dass sich der Strömungsweg des Fluids durch die Zuführungsleitung in einem Übergangsabschnitt der Zuführungsleitung von dem ersten Abschnitt auf den zweiten Abschnitt trichterförmig in Richtung zu der Expansionskammer verjüngt. Mit dieser trichterartigen Verjüngung des Innenquerschnitts der Zuführungsleitung in dem Übergangsabschnitt kann ein gestufter Verlauf des Strömungsquerschnitts entlang der Zuführungsleitung mit einer in dem Verjüngungsbereich vorzugsweise kontinuierlichen (stetigen) oder schrittweisen Abnahme des Innenquerschnitts geschaffen werden. Dadurch, dass sich der Innenquerschnitt der Zuführungsleitung zumindest einmal trichterförmig in Strömungsrichtung des Fluids durch die Zuführungsleitung in Richtung zu der Expansionskammer verjüngt, wird das Fluid in dem zumindest einen trichterförmigen Übergangsabschnitt der Zuführungsleitung beschleunigt. Auf Grund der trichterförmigen Verjüngung in dem Übergangsabschnitt nimmt der Strömungsquerschnitt nicht sprunghaft (abrupt) von dem Strömungsquerschnitt des ersten Abschnitts auf den im Vergleich zu dem Strömungsquerschnitt des ersten Abschnitts kleineren Strömungsquerschnitt des zweiten Abschnitts ab. Damit können auf Grund der Trichterform des Übergangsabschnitts Druckschwankungen des beschleunigten Fluids in dem auf den trichterförmigen Übergangsabschnitt folgenden Abschnitt der Zuführungsleitung weitgehend reduziert oder vermieden werden.

Das erfindungsgemäße Instrument arbeitet zur Abkühlung des Arbeitsabschnitts des Instruments mit dem Joule-Thomson-Effekt, der sich an dem Fluid bei der Expansion des Fluids in der Expansionskammer zeigt. Durch die gleichmäßige Beschleunigung in dem Übergangsabschnitt und die Verwendung des Kapillarleitungsabschnitts als distalen Endabschnitt der Zuführungsleitung wird erreicht, dass die Strecke, über die die Fluidteilchen nach dem Austritt aus der Mündungsöffnung in die Expansionskammer weitgehend zusammenbleiben, gegenüber einem Instrument, das eine beschriebene trichterförmige Verjüngung und einen Kapillarleitungsabschnitt nicht aufweist, verlängert ist. Damit kann insbesondere vermieden werden, dass sich der Strahl direkt nach der Mündung übermäßig aufweitet und damit den Rückstrom des Gases aus der Expansionskammer behindert. Dadurch kann der Instrumentenkopf, der die Expansionskammer enthält und zumindest einen Abschnitt der Rückführungseinrichtung enthalten kann, schlank ausgebildet werden. Dies ebnet den Weg zu miniaturisierten Instrumentenköpfen. Die Verwendung der Kapillarleitung als Drossel für das Fluid sowie die weitgehend druckstoßfreie Beschleunigung des Fluids in dem wenigstens einen Übergangsabschnitt, in dem sich der Strömungsquerschnitt trichterförmig verjüngt, ebnen insbesondere den Weg zu einem besonders schlanken Instrumentenkopf, mit dem beispielsweise eine sichere Gewebeprobenentnahme vereinfacht sein kann.

Besonders bevorzugt ist die Zuführungsleitung derart ausgebildet, dass sich der Innenquerschnitt der Zuführungsleitung in dem Übergangsabschnitt auf den Kapillarleitungsabschnitt trichterförmig verjüngt. Dadurch wird das Fluid zum einen beschleunigt und zum anderen können Druckschwankungen beim Eintritt in den Kapillarleitungsabschnitt weitgehend reduziert oder vermieden werden, was zu einer großen freien Weglänge des Fluids führt, über die die Fluidteilchen nach dem Austritt aus dem Kapillarleitungsabschnitt in die Expansionskammer weitgehend zusammenbleiben. Vorzugsweise ist die Verjüngung des Innenquerschnitts in einem Übergangsbereich, der sich von vor dem Übergangsabschnitt auf den Kapillarleitungsabschnitt, durch den Übergangsabschnitt und bis in den Kapillarleitungsabschnitt erstreckt, kontinuierlich. Die Innenwandfläche der Zuführungsleitung in dem Übergangsbereich ist vorzugsweise kantenfrei, so dass innerhalb des Übergangsbereichs entlang des Strömungsweges keine sprunghaften Veränderungen des Gradienten des Innenquerschnitts vorhanden sind.

Vorzugsweise beträgt der Verjüngungswinkel, mit dem sich der Innenquerschnitt der Zuführungsleitung zumindest in dem Übergangsabschnitt auf den Kapillarleitungsabschnitt trichterartig verjüngt, zwischen minimal 15° und maximal 40°. Der Verjüngungswinkel wird von gegenüberliegenden Abschnitten der Innenwandfläche des Übergangsabschnitts eingeschlossen, die den Strömungsquerschnitt durch den Übergangsabschnitt bestimmt.

Die Länge des Kapillarleitungsabschnitts beträgt vorzugsweise zwischen minimal 1 mm bis maximal 15 mm. Der Innendurchmesser des Kapillarleitungsabschnitts, der den Strömungsquerschnitt des Kapillarleitungsabschnitts bestimmt, beträgt vorzugsweise zwischen minimal 60 Mikrometer bis maximal 200 Mikrometer.

Vorzugsweise weist die Zuführungsleitung wenigstens zwei Übergangsabschnitte auf, in denen sich der Strömungsweg durch die Zuführungsleitung in Strömungsrichtung zu der Expansionskammer trichterförmig verjüngt.

Der erste Abschnitt und der zweite Abschnitt bilden vorzugsweise Stufenabschnitte einer Reihe von zwei, drei oder mehr als drei Stufenabschnitten der Zuführungsleitung, wobei zwischen je zwei Stufenabschnitten ein Übergangsabschnitt angeordnet ist, an den die beiden Stufenabschnitte angrenzen. Wie beschrieben nimmt der Strömungsquerschnitt durch wenigstens einen Übergangsabschnitt, bevorzugt in jedem der Übergangsabschnitte, wie bei einem Trichter in Richtung zu der Mündungsöffnung der Zuführungsleitung zu der Expansionskammer ab. Die Flächeneinhalte der Innenquerschnittsflächen jedes Stufenabschnitts gehören einer Innenquerschnittstufe an, wobei die Flächeninhalte der Innenquerschnittsflächen einer Innenquerschnittsstufe eines Stufenabschnitts größer sind als die Flächeninhalte der Innenquerschnittsflächen der Innenquerschnittsstufe des in Richtung zu der Mündung des Kapillarleitungsabschnitts stromabwärts an denselben Übergangsabschnitt angrenzenden Stufenabschnitts. Dadurch wird ein gestufter Verlauf des Strömungsquerschnitts der Zuführungsleitung bis zu der Mündung der Zuführungsleitung geschaffen, wobei sich der Strömungsweg in den Übergangsabschnitten mit trichterförmiger Verjüngung auf Grund der Trichterform nicht abrupt von einer Querschnittsstufe auf die folgende Querschnittsstufe, sondern bevorzugt weitgehend kontinuierlich oder schrittweise oder in wenigstens einem Längsabschnitt des Übergangsabschnitts kontinuierlich und in wenigstens einem anderen Längsabschnitt des Übergangsabschnitts schrittweise in Richtung zu der Expansionskammer verjüngt und in den Stufenabschnitten entlang der Stufenabschnitte jeweils vorzugsweise weitgehend konstant bleiben kann. Der Kapillarleitungsabschnitt kann den in Strömungsrichtung zu der Mündung letzten Stufenabschnitt der Reihe bilden. Durch die Beschleunigung in den trichterförmigen Übergangsabschnitten erreichen die Fluidteilchen eine hohe Geschwindigkeit, die den Fluidstrahl nach dem Austritt aus der Mündung weit in die Expansionskammer trägt, wodurch der Expansionsbereich des Fluids vergrößert wird und dadurch die Effektivität der Abkühlung verbessert werden kann. Aufgrund der trichterartigen Verjüngung und der angeordneten Stufenabschnitte erfolgt die Beschleunigung des Fluides in Richtung zu der Expansionskammer über den Verlauf der Reihe gesehen schrittweise, wobei dadurch eine Reduzierung von Druckstößen und Turbulenzen in dem Fluid erreicht werden kann. Aufgrund dessen ist der Bereich in der Expansionskammer vergrößert, in dem das Gas expandiert.

Der Strömungsquerschnitt für das Fluid steigt beim Übergang von der Mündungsöffnung des Kapillarleitungsabschnitts in die Expansionskammer vorzugsweise sprunghaft an. Dies fördert eine kräftige Ausbildung des Joule-Thompson-Effekts an dem expandierenden Fluid. Zudem kann ein Abschnitt der Expansionskammer als Teil der Rückführungseinrichtung zur Verfügung stehen.

Die Zuführungsleitung ist vorzugsweise in der Rückführungsleitung angeordnet und/oder die Rückführungsleitung ist beispielsweise neben der Zuführungsleitung angeordnet. Besonders bevorzugt ist das Verhältnis des Strömungsquerschnitts in der Rückführungsleitung neben dem Kapillarleitungsabschnitt und/oder um den Kapillarleitungsabschnitt herum zu dem Innenquerschnitt des Kapillarleitungsabschnitts größer oder gleich 5.

Erfindungsgemäss ist die Zuführungsleitung derart ausgebildet, dass der Außenquerschnitt (Außenquerschnittsfläche) der Zuführungsleitung an den trichterförmigen Übergangsabschnitten nicht sprunghaft von dem Außenquerschnitt eines Stufenabschnitts auf den Außenquerschnitt des an denselben Übergangsabschnitt angrenzenden Stufenabschnitts abnimmt, sondern vorzugsweise kontinuierlich oder schrittweise oder in wenigstens einem Unterabschnitt des Abschnitts der Zuführungsleitung, dessen Außenquerschnitt sich verjüngt, schrittweise und in wenigstens einem anderen Unterabschnitt des Abschnitts kontinuierlich in Richtung zu der Mündung des Kapillarleitungsabschnitts abnimmt. In Strömungsrichtung des von der Expansionskammer wegströmenden Gases nach der Expansion gesehen nimmt der Außenquerschnitt der Zuführungsleitung an den trichterförmigen Übergangsabschnitten entsprechend vorzugsweise nicht sprunghaft, sondern vorzugsweise kontinuierlich und/oder schrittweise zu. Wenn die Wand der Zuführungsleitung gleichzeitig eine Wand der Rückführungseinrichtung, insbesondere einer Rückführungsleitung, bildet, kann die Rückführung des Gases aus dem Expansionsbereich durch den durch die Außenquerschnittsreduktion bereitgestellten Raum verbessert sein. Anders als bei einer sprunghaften Abnahme des Außenquerschnitts der Zuführungsleitung in Richtung zu der Mündung, wird bei der kontinuierlichen Abnahme oder bei der schrittweisen Abnahme des Außenquerschnitts der Strömungsquerschnitt für das rückströmende Gas nicht abrupt verändert, beispielsweise verjüngt. Dadurch kann der Strömungswiderstand der Rückführungseinrichtung, insbesondere einer Rückführungsleitung, erniedrigt sein.

Das Instrument kann derart ausgebildet sein, dass der Strömungsquerschnitt der Rückführungsleitung in Strömungsrichtung des Gases bei der Rückführung weg von der Expansionskammer an den Übergangsabschnitten kontinuierlich oder schrittweise oder an den Übergangsabschnitten in wenigstens einem Längsabschnitt kontinuierlich und in wenigstens einem anderen Längsabschnitt schrittweise abnimmt.

Bevorzugt ist zumindest der Abschnitt der Zuführungsleitung mit dem Kapillarleitungsabschnitt und dem an den Kapillarleitungsabschnitt angrenzenden Übergangsabschnitt nahtlos einstückig ausgebildet. Dies vereinfacht die prozesssichere Herstellung des Instruments zur Vermeidung von Störungen und abrupten Änderungen des Strömungsquerschnitts der Zuführungsleitung hin zu deren Mündung. Besonders bevorzugt ist zumindest der Abschnitt der Zuführungsleitung mit dem Kapillarleitungsabschnitt und den trichterförmigen Übergangsabschnitten nahtlos einstückig ausgebildet, so dass die prozesssichere Herstellung der Übergangsabschnitte und des Kapillarleitungsabschnitts vereinfacht ist.

Die Zuführungsleitung insgesamt kann mit einem Rundknetverfahren hergestellt sein. Vorzugsweise ist zumindest der Abschnitt der Zuführungsleitung mit dem Kapillarleitungsabschnitt und dem an den Kapillarleitungsabschnitt angrenzenden Übergangsabschnitt mit dem Rundknetverfahren hergestellt. Besonders bevorzugt ist zumindest der Abschnitt der Zuführungsleitung mit dem Kapillarleitungsabschnitt und den trichterförmigen Übergangsabschnitten mit dem Rundknetverfahren hergestellt. Mit dem Rundknetverfahren lässt sich eine hohe Güte mit geringer Oberflächenrauigkeit und geringer Oberflächenwelligkeit der den Strömungsquerschnitt bestimmenden Innenfläche der Zuführungsleitung prozesssicher erzielen.

Die Wanddicke des Kapillarleitungsabschnitts kann gleich oder geringer sein als die Wandstärke des Zuführungsleitungsabschnitts, der stromaufwärts an den Übergangsabschnitt auf den Kapillarleitungsabschnitt angrenzt. Dies erleichtert die Bereitstellung eines großen Raumes neben dem Kapillarleitungsabschnitt oder um den Kapillarleitungsabschnitt herum für die Rückführung des Gases aus der Expansionszone. Zudem kann dadurch der Wärmeübergang zwischen dem neben oder um den Kapillarleitungsabschnitt herum zurückgeführten Gas und dem durch den Kapillarleitungsabschnitt zugeführten Gas vergrößert werden.

Das Verhältnis des Innendurchmessers des Kapillarleitungsabschnitts zu der Länge des Kapillarleitungsabschnitts beträgt vorzugsweise zwischen minimal 0,004 bis maximal 0,2.

Vorzugsweise ist die Mündungsöffnung des Kapillarleitungsabschnitts, durch die das Fluid aus der Zuführungsleitung austritt und in die Expansionskammer eintritt, an der Stirnseite des Kapillarleitungsabschnitts angeordnet. Vorzugsweise ist der Mantel des Kapillarleitungsabschnitts, der das Lumen des Kapillarleitungsabschnitts umschließt, durch das das Fluid strömt, frei von seitlichen Öffnungen.

Der Abstand zwischen der Mündungsöffnung und der gegenüberliegenden Wandfläche der Expansionskammer, die das Lumen der Expansionskammer begrenzt, beträgt vorzugsweise zwischen minimal 0,5 mm und maximal 5 mm.

Weitere vorteilhafte Merkmale des erfindungsgemäßen kryochirurgischen Instruments ergeben sich aus den Unteransprüchen sowie folgender Beschreibung und den Figuren.

Es zeigen:
Figur 1 - ein distales Ende eines kryochirurgischen Instruments des Standes der Technik in einer ausschnittsweisen Längsschnittansicht,
Figur 2a - eine ausschnittsweise Ansicht eines beispielhaften erfindungsgemäßen kryochirurgischen Instruments in einer Längsschnittdarstellung,
Figuren 2b bis 2d - Querschnittansichten des in Figur 2a dargestellten erfindungsgemäßen Instruments an den in Figur 2a eingetragenen Schnittebenen,
Figur 3 - ein Ausschnitt eines beispielhaften erfindungsgemäßen kryochirurgischen Instruments in einer Längsschnittdarstellung,
Figur 4 - ein Ausschnitt eines erfindungsgemäßen kryochirurgischen Instruments gemäß einer weiteren beispielhaften Ausführungsform in einer Längsschnittdarstellung,
Figur 5 - eine ausschnittweise Längsschnittdarstellung eines beispielhaften erfindungsgemäßen kryochirurgischen Instruments geführt in dem Arbeitskanal eines Endoskops,
Figur 6 - eine ausschnittsweise Längsschnittdarstellung eines beispielhaften erfindungsgemäßen Instruments und
Figur 7 - eine ausschnittweise Längsschnittdarstellung eines beispielhaften erfindungsgemäßen Instruments.

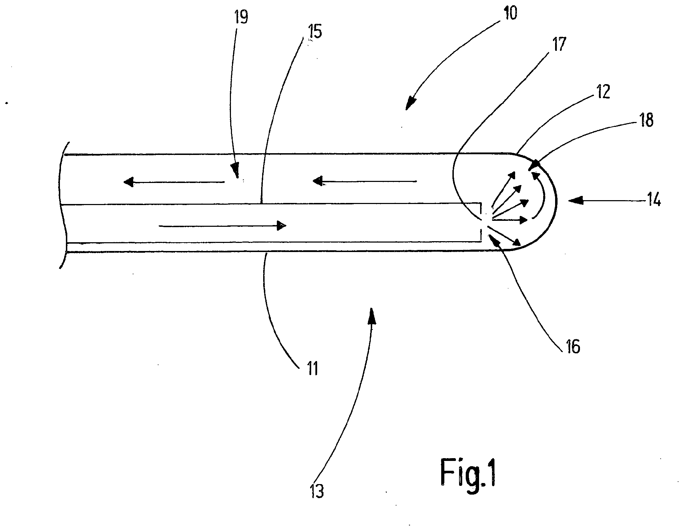

Figur 1 zeigt einen distalen Endabschnitt 13 eines kryochirurgischen Instruments 10 des Standes der Technik in einer Längsschnittdarstellung. Das Instrument 10 weist einen Schaft 11 auf, der sich bis zu einem Kopf 12 des Instruments 10 am distalen Ende 13a des Instruments 10 erstreckt. Außen an dem Kopf 12 ist eine Anhaftungsfläche 14 vorgesehen, an der eine Gewebeprobe zur Entnahme festgefroren werden kann. Innerhalb des Schaftes 11 ist eine Zuführungsleitung 15 zum Zuführen von Gas an das distale Ende 13a des Instruments 10 angeordnet. Die Zuführungsleitung 15 endet mit einer Drosselblende 16 mit einer Öffnung (Mündung) 17, durch die das Gas aus der Zuführungsleitung 15 in eine Expansionskammer 18 im Kopf 12 des Instruments 10 strömen kann. Wenn der Gasstrom aus der Zuführungsleitung 15 an der Drosselblende 16 gedrosselt wird, und sich das Gas nach der Drosselblende 16 nach dem Eintritt in die Expansionskammer 18 entspannt, zeigt sich an dem Gas der Joule-Thomson-Effekt, indem das in der Expansionskammer 18 entspannende Gas eine Temperaturverminderung erfährt. Dadurch kann dieses den Kopf 12 des Instruments 10 mit der Anhaftungsfläche 14 abkühlen. Das abgekühlte Gas verlässt die Expansionskammer 18 durch eine Rückführungsleitung 19, die in dem Schaft 11 neben der Zuführungsleitung 15 angeordnet ist. Der Rückfluss des Gases aus der Expansionskammer 18 in die Rückführungsleitung 19 kann, wie durch die Pfeile in Figur 1 angedeutet, von dem aus der Mündungsöffnung 17 ausströmenden Gas behindert werden. Es muss daher eine relativ große Expansionskammer 18 bereitgestellt werden, um einen geeigneten Rückfluss gewährleisten zu können.

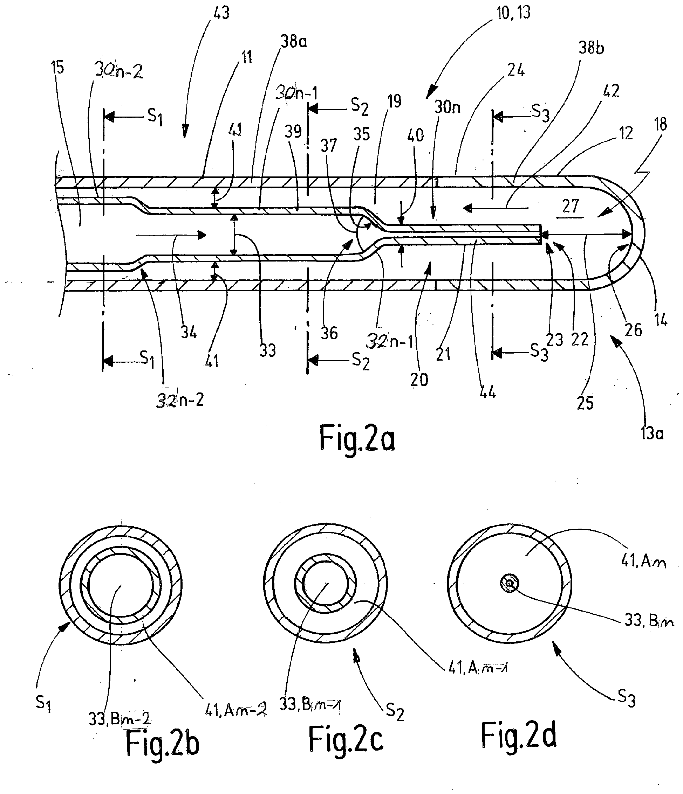

Figur 2a zeigt ein erfindungsgemäßes kryochirurgisches Instrument 10 in einer Längsschnittdarstellung. Bei dem erfindungsgemäßen kryochirurgischen Instrument 10 wird das distale Ende 20 der Zuführungsleitung 15 von einem Kapillarleitungsabschnitt 21 (Kapillarrohrabschnitt) gebildet. Der Kapillarleitungsabschnitt 21 weist eine Mündung 22 in die Expansionskammer 18 an der Stirnseite 23 des Kapillarleitungsabschnitts 21 auf. Der Kapillarleitungsabschnitt 21 reicht bis in den Kopf 12 des Instruments 10, der von einer Kappe 24 gebildet wird, die die Expansionskammer 18 umgrenzt. Der Abstand 25 zwischen der Mündungsöffnung 22 des Kapillarleitungsabschnitts 21 und der gegenüberliegenden Wandfläche 26 der Kappe 24, die die Expansionskammer 18 begrenzt, beträgt vorzugsweise minimal 0,5 mm bis maximal 5 mm. Die der Mündung 22 des Kapillarrohrabschnitts 21 gegenüberliegende, das Lumen 27 der Expansionskammer 18 begrenzende Wandfläche 26 der Kappe 24 kann, wie dargestellt, beispielsweise eine Kugelkappenfläche 26 sein, die dazu eingerichtet und angeordnet ist, das auf die Wandfläche 26 der Kappe 24 auftreffende Gas in die Rückführungsleitung 19 zu leiten.

Der Kapillarleitungsabschnitt 21 bildet den n-ten Stufenabschnitt 30n einer Reihe von wenigstens n=2, bevorzugt n>2, beispielsweise, und wie in Figur 2a dargestellt, n=3 Stufenabschnitten 30n-2, 30n-1, 30n der Zuführungsleitung 15. Zwischen zwei Stufenabschnitten 30n-2, 30n-1, 30n ist jeweils ein Übergangsabschnitt 32n-2, 32n-1 angeordnet, an den die beiden Stufenabschnitte 30n-2, 30n-1 bzw. 30n-1, 30n angrenzen. In zumindest einem Übergangsabschnitt 32n-2, 32n-1 nimmt die Innenquerschnittsfläche 33 der Zuführungsleitung 15 in distaler Richtung 34 zu der Mündung 22 des Kapillarleitungsabschnitts 21 vorzugsweise trichterförmig, beispielsweise konisch, ab, so dass es bei Beaufschlagung des Instruments 10 mit einem Fluid, z.B. einem Gas in den Übergangsabschnitten 32n-2, 32n-1 zu einer Beschleunigung des durch die Zuführungsleitung 15 zu der Mündung 22 strömenden Fluids kommt. Die innere Wandfläche 35 des an den Kapillarrohrabschnitt 21 angrenzenden Übergangsabschnitts 32n-1 weist vorzugsweise im Wesentlichen keine senkrecht zur Strömungsrichtung 34 des Gases gestellten Flächenabschnitte auf, gegen die das durch den Übergangsabschnitt in Strömungsrichtung 34 zu der Expansionskammer 18 strömende Gas strömen müsste. Dasselbe gilt vorzugsweise auch für jeden der übrigen Übergangsabschnitte 32n-1. Vielmehr weist der dargestellte beispielhafte Übergangsabschnitt 32n-1 auf den Kapillarrohrabschnitt 21 eine im Längsschnitt durch den Übergangsabschnitt 32n-1 gesehen zur Strömungsrichtung 34 schräggestellte Innenwandfläche 35 auf, deren Umfangsabschnitte mit der Strömungsrichtung 34 spitze Winkel kleiner 90° einschließen. Die übrigen Übergangsabschnitte 32n-2 sind vorzugsweise ebenso ausgebildet. Fig. 2a zeigt einen trichterförmigen Übergangsabschnitt 32n-2 auf den vorletzten Stufenabschnitt 30n-1 und einen trichterförmigen Übergangsabschnitt 32n-1 auf den Kapillarleitungsabschnitt 30n, der den letzten Stufenabschnitt 30n der Reihe bildet. Vorzugsweise verjüngt sich der Strömungsweg in einem Übergangsbereich 36 von vor dem Übergangsabschnitt 32n-1 auf den Kapillarleitungsabschnitt 30n, 21, durch den Übergangabschnitt 32n-1, in den Kapillarleitungsabschnitt 21 kontinuierlich. Vorzugsweise sind in dem Übergangsbereich 36 in dem Strömungsweg in der Zuführungsleitung 15 insbesondere im Wesentlichen keine senkrecht zur Strömungsrichtung 34 gestellten Innenwandflächen der Zuführungsleitung 15 vorhanden, die zu einer sprunghaften Änderung des Strömungsquerschnitts führen würden. Vorzugsweise nimmt der Strömungsquerschnitt der Zuführungsleitung 15 in jedem Übergangsabschnitt 32n-2, 32n-1 der Zuführungsleitung 15 zwischen den Stufenabschnitten 30n-2, 30n-1, 30n trichterförmig in Strömungsrichtung 34 in Richtung Mündung 22 ab, so dass vorzugsweise eine Reihe aus abwechselnd angeordneten Stufenabschnitten 30n-2, 30n-1, 30n und Übergangsabschnitten 32n-2, 32n-1 mit trichterförmiger Innenquerschnittsverjüngung gebildet ist.

Es ist vorteilhaft, wenn der Strömungsquerschnitt in dem oder den Übergangsabschnitten 32n-2, 32n-1 der Zuführungsleitung 15 jeweils nicht sprunghaft von dem Strömungsquerschnitt in dem Stufenabschnitt 30n-2 bzw. 30n-1 der Zuführungsleitung 15, der vor dem Übergangsabschnitt 32n-2 bzw. 32n-1 angeordnet ist und an den Übergangsabschnitt 32n-2 bzw. 32n-1 angrenzt, auf den Strömungsquerschnitt in dem Stufenabschnitt 30n-1 bzw. 30n der Zuführungsleitung 15 abnimmt, der in Strömungsrichtung 34 nach dem Übergangsabschnitt 32n-2 bzw. 32n-1 an den Übergangsabschnitt 32n-2 bzw. 32n-1 angrenzt, sondern wenn sich der Strömungsweg in dem oder den Übergangsabschnitten 32n-2, 32n-1 jeweils über einen Wegabschnitt des Strömungswegs hinweg in Richtung zu der Mündung 22 verjüngt. Denn dies reduziert Verwirbelungen des Fluids und Druckschwankungen des Fluids in dem auf den Übergangsabschnitt 32n-2 bzw. 32n-1 folgenden Stufenabschnitt 30n-1, 30n der Zuführungsleitung 15.

Der Verjüngungswinkel 37 des Innenquerschnitts 33 in dem Übergangsabschnitt 32n-1 auf den Kapillarrohrabschnitt 21, 30n beträgt vorzugsweise minimal 15° bis maximal 40°. Der Verjüngungswinkel 37 wird durch die innere Wandfläche 35 des Übergangsabschnitts 32n-1 festgelegt, die den Strömungsquerschnitt durch den Übergangsabschnitt 32n-1 seitlich begrenzt. Die innere Wandfläche 35 der Übergangsabschnitte 32n-2, 32n-1 ist im Längsschnitt durch die Zuführungsleitung 15 entlang der Strömungsrichtung 34 gesehen vorzugsweise zur Strömungsrichtung 34 schräggestellt angeordnet. Die innere Wandfläche 35 kann beispielsweise eine Kegelstumpfmantelfläche oder eine Pyramidenstumpfmantelfläche sein. Der Übergangsabschnitt 32n-2 auf den vorletzten Stufenabschnitt 30n-1 und/oder der Übergangsabschnitt 32n-1 auf den Kapillarleitungsabschnitt 21 können symmetrisch bzgl. einer Ebene parallel zur Strömungsrichtung 34 sein. Die Mittelpunkte der Strömungsquerschnittsflächen in dem Übergangsabschnitt 32n-2 auf den vorletzten Stufenabschnitt 30n-1 und/oder die Mittelpunkte der Strömungsquerschnittsflächen in dem Übergangsabschnitt 32n-1 auf den Kapillarleitungsabschnitt 21 können, wie bei einem symmetrischen Trichter, auf einer geraden Linie liegen, die senkrecht zu der Strömungsquerschnittsfläche in dem Eingang in den betreffenden Übergangsabschnitt 32n-1, 32n-2 steht. Alternativ zu einer symmetrisch trichterförmigen Verjüngung des Strömungsquerschnitts in einem oder mehreren Übergangsabschnitten 32n-2, 32n-1 kann sich der Strömungsquerschnitt des Übergangsabschnitts 32n-2 auf den vorletzten Stufenabschnitt 30n-1 und/oder des Übergangsabschnitts 32n-1 auf den letzten Stufenabschnitt 30n beispielsweise wie bei einem asymmetrischen Trichter verjüngen.

Die Stufenabschnitte 30n-2, 30n-1, 30n legen Innenquerschnittsstufen fest. In einem Stufenabschnitt 30n-2, 30n-1, 30n gehören die Innenquerschnitte einer Innenquerschnittstufe an. Innerhalb jedes Stufenabschnitts 30n-2, 30n-1, 30n bleibt der Innenquerschnitt der Zuführungsleitung 15 innerhalb eines bestimmten Wertebereichs (Stufe). Innerhalb eines Stufenabschnitts 30n-1, 30n kann der Strömungsquerschnitt beispielsweise konstant sein. Die Innenquerschnitte in dem Wertebereich eines Stufenabschnitts 30n-2, 30n-1, sind größer als die Innenquerschnitte in dem Wertebereich des jeweils stromabwärts (hin zu der Mündung) nachfolgenden Stufenabschnitts 30n. Die Zuführungsleitung 15 weist entsprechend einen gestuften Verlauf des Innenquerschnitts mit zwischen den Stufen in den Übergangsabschnitten 32n-2, 32n-1 nicht sprunghaftem, sondern vorzugsweise kontinuierlichem oder schrittweisem Übergang des Strömungsquerschnitts auf die nächste Stufe auf. Es ist auch möglich, dass sich der Strömungsquerschnitt in zumindest einem Übergangsabschnitt 32n-2, 32n-1 in wenigstens einem ersten Längsabschnitt des Übergangsabschnitts 32n-2, 32n-1 schrittweise und in wenigstens einem anderen Längsabschnitt des Übergangsabschnitts 32n-2, 32n-1, der stromaufwärts oder stromabwärts des ersten Längsabschnitts angeordnet ist, kontinuierlich verjüngt, so dass sich der Strömungsquerschnitt in dem Übergangsabschnitt 32n-2, 32n-1 insgesamt kontinuierlich und schrittweise auf die nächste Stufe verjüngt. Die Zuführungsleitung 15 kann insbesondere derart ausgebildet sein, dass der Innenquerschnitt der Zuführungsleitung 15 von dem Anfang der Reihe aus Stufenabschnitten 30n-2, 30n-1, 30n in Strömungsrichtung 34 bis zu der Mündung 22 der Zuführungsleitung 15 monoton abnimmt. Dies bedeutet, dass der Innenquerschnitt zumindest abschnittsweise streng monoton abnimmt und gegebenenfalls abschnittsweise gleich bleiben kann.

In einer Ausführungsform kann die Innenwandfläche 35 der Zuführungsleitung 15 in dem Übergangsabschnitt 30n-1 auf den Kapillarleitungsabschnitt 21 in den Kapillarleitungsabschnitt 21 hinein bis zu der Mündung 22 der Zuführungsleitung 15 frei von quer zur Strömungsrichtung 34 durch die Zuführungsleitung 15 orientierten Kanten oder Knicken sein, die eine sprunghafte Änderung des Gradienten des Strömungsquerschnitts der Zuführungsleitung 15 bedeuten würden.

Neben der Zuführungsleitung 15 und/oder um die Zuführungsleitung 15 herum ist vorzugsweise der Strömungsquerschnitt der Rückführungsleitung 19 ausgebildet. Im dargestellten Ausführungsbeispiel ist die Zuführungsleitung 15 zumindest abschnittsweise in der Rückführungsleitung 19 angeordnet. Der Strömungsquerschnitt der Rückführungsleitung 19 ist einerseits durch die Wand 38a des Schaftes sowie die Wand 38b des Kopfes 12 und andererseits durch die Wand 39 der Zuführungsleitung 15 begrenzt. Die Zuführungsleitung 15 ist in der Figur 2a als koaxial in dem Schaft 11 und der Kappe 24 angeordnet dargestellt. Die Zuführungsleitung 15 sowie der Schaft 11 und/oder die Kappe 24 können jedoch stattdessen nicht-koaxial, vorzugsweise mit parallelen Mittelachsen, sein.

Erfindungsgemäss nimmt der Außenquerschnitt 40 der Zuführungsleitung 15 an den Übergangsabschnitten 32n-2, 32n-1, wie dargestellt, in Richtung 34 zu der Mündung 22 nicht sprunghaft, sondern vorzugsweise kontinuierlich oder schrittweise ab. An zumindest einem Übergangsabschnitt 32n-2, 32n-1 kann der Außenquerschnitt 40 der Zuführungsleitung 15 längsabschnittsweise kontinuierlich und längsabschnittsweise schrittweise in Richtung zu der Mündung 22 abnehmen. Dadurch kann der Strömungsquerschnitt 41 der Rückführungsleitung 19, wie in dem Ausführungsbeispiel gemäß Figur 2a gezeigt, an den Übergangsabschnitten 32n-2, 32n-1 in Richtung 42 des durch die Rückführungsleitung 19 von der Expansionskammer 18 wegströmenden Gases jeweils über die Länge der Übergangsabschnitte abnehmen, also nicht sprunghaft von dem Strömungsquerschnitt vor dem Übergangsabschnitt 32n-2, 32n-1 auf den Strömungsquerschnitt nach diesem Übergangsabschnitt 32n-2, 32n-1. Der Strömungsquerschnitt 41 der Rückführungsleitung 19 kann in Strömungsrichtung 42 des von der Expansionskammer 18 wegströmenden Gases an den Übergangsabschnitten 32n-2, 32n-1 insbesondere kontinuierlich oder schrittweise oder längsabschnittsweise kontinuierlich und längsabschnittsweise schrittweise abnehmen. Der Strömungsquerschnitt 41 der Rückführungsleitung 19 neben dem Kapillarrohrabschnitt 21, 30n oder um den Kapillarrohrabschnitt 21, 30n herum und/oder zwischen den Übergangsabschnitten 32n-2, 32n-1 kann weitgehend konstant sein.

Die Stufenabschnitte 30n-2, 30n-1, 30n legen vorzugsweise Außenquerschnittsstufen fest. An einem Stufenabschnitt 30n-2, 30n-1, 30n gehören die Außenquerschnitte (Außenquerschnittsflächen) der Zuführungsleitung 15 einer Außenquerschnittstufe an. Innerhalb jedes Stufenabschnitts bleibt der Außenquerschnitt der Zuführungsleitung innerhalb eines bestimmten Wertebereichs (Stufe). Entlang eines Stufenabschnitts 30n-2, 30n-1, 30n können die Außenquerschnitte des Stufenabschnitts 30n-2, 30n-1, 30n beispielsweise konstant sein. Die Außenquerschnitte in dem Wertebereich eines Stufenabschnitts 30n-2, 30n-1 sind größer als die Außenquerschnitte in dem Wertebereich des jeweils stromabwärts (hin zu der Mündung) nachfolgenden Stufenabschnitts 30n-1, 30n. Die Zuführungsleitung 15 weist entsprechend vorzugsweise einen gestuften Verlauf des Außenquerschnitts mit zwischen den Stufen an den Übergangsabschnitten 32n-2, 32n-1nicht sprunghaftem Übergang des Außenquerschnitts auf die nächste Stufe auf. Vielmehr erstreckt sich der Übergang vorzugsweise jeweils über die Länge des Übergangsabschnitts 32n-2, 32n-1 und/oder ist der Übergang des Außenquerschnitts auf die nächste Stufe vorzugsweise kontinuierlich oder erfolgt, aus Sicht des strömenden Fluids, schrittweise. Der Außenquerschnitt der Zuführungsleitung 15 ist zwischen den in Figur 2c dargestellten Übergangsabschnitten 32n-2, 32n-1 und zwischen dem Übergangsabschnitt 32n-1 auf den Kapillarleitungsabschnitt 21 und der Mündung 22 vorzugsweise weitgehend konstant, so dass der Kapillarleitungsabschnitt 21 einen über die Längserstreckung des Kapillarleitungsabschnitts 21 weitgehend konstanten Außenquerschnitt aufweist.

Wie anhand der Figuren 2b bis 2d gezeigt, nimmt in dem in Figur 2a dargestellten Ausführungsbeispiel durch die Ausbildung der Zuführungsleitung 15 in dem Schaft 11 das Verhältnis des Strömungsquerschnittsflächeninhalts 41 (An-2, An-1, An) der Rückführungsleitung 19 neben einem Stufenabschnitt 30n-2, 30n-1, 30n oder um einen Stufenabschnitt 30n-2, 30n-1, 30 n herum zu dem Innenquerschnittsflächeninhalt 33 (Bn-2, Bn-1, Bn) in dem Stufenabschnitt 30 in Strömungsrichtung 34 zu der Mündung 22 von Stufenabschnitt zu Stufenabschnitt zu, ist also an dem Kapillarleitungsabschnitt 21 am größten. Es gilt entsprechend An:Bn>An-1:Bn-1>An-2:Bn-2.

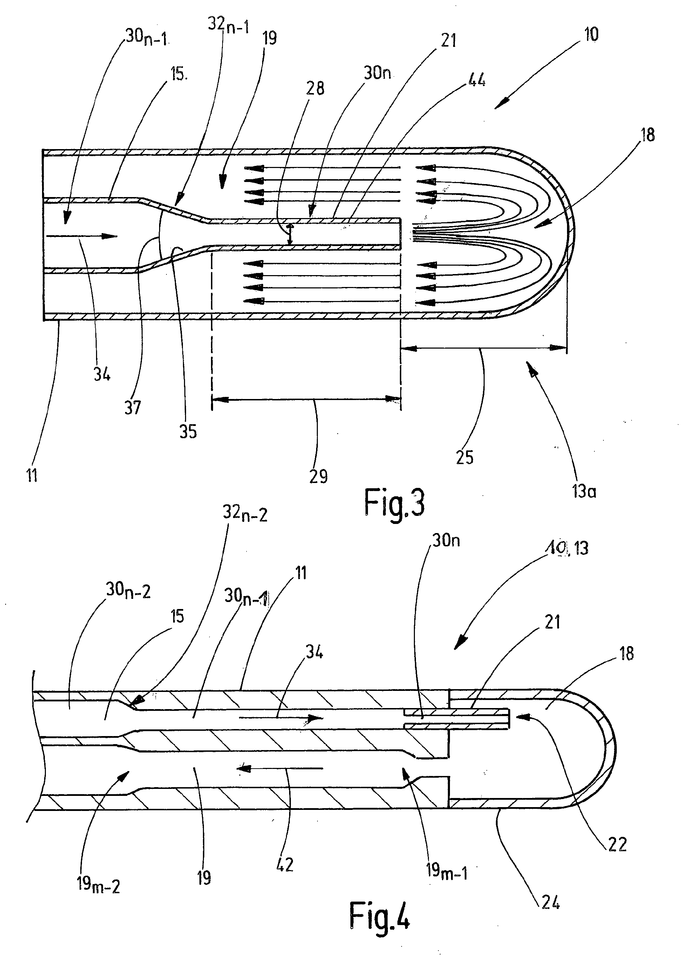

Das Verhältnis des Flächeninhalts des Strömungsquerschnitts der Rückführungsleitung 19 neben dem Kapillarleitungsabschnitt 21 und/oder um den Kapillarleitungsabschnitt 21 herum zu dem Flächeninhalt des Strömungsquerschnitts des Kapillarleitungsabschnitts 21 ist vorzugsweise größer oder gleich 5. Der Innendurchmesser 28 (der Übersichtlichkeit halber beispielhaft in Figur 3 eingezeichnet) des Kapillarleitungsabschnitts bestimmt den Strömungsquerschnitt 33 des Kapillarleitungsabschnitts. Das Verhältnis des Innendurchmessers 28 des Kapillarleitungsabschnitts 21 zu der Länge 29 (der Übersichtlichkeit halber beispielhaft in Figur 3 eingezeichnet)des Kapillarleitungsabschnitts 21 beträgt vorzugsweise zwischen minimal 0,004 bis maximal 0,2. Die Länge 29 des Kapillarrohrs, das den Kapillarleitungsabschnitt 21 bildet, kann beispielsweise zwischen minimal 1 mm bis maximal 15 mm betragen. Der Innendurchmesser 28 des Kapillarleitungsabschnitts 21 kann beispielsweise minimal 60 Mikrometer bis maximal 200 Mikrometer betragen.

Der Abschnitt der Zuführungsleitung 15 mit den Übergangsabschnitten 32n-2, 32n-1, dem Stufenabschnitt 30n-1 zwischen den Übergangsabschnitten 32n-2, 32n-1 und dem Kapillarleitungsabschnitt 21, 30n ist vorzugsweise nahtlos einstückig ausgebildet. Der Abschnitt kann beispielsweise mit Hilfe des Rundknetverfahrens hergestellt sein. Die den Kopf 12 bildende Kappe 24 des Schaftes 11 mit der Anhaftungsfläche 14 kann bspw. aus Edelstahl bestehen. Der Schaft 11 kann bspw. aus PEEK, PA, PUR oder PTFE bestehen. Der Schaft 11 kann starr oder flexibel sein.

Beim Betrieb des kryochirurgischen Instruments 10 geschieht Folgendes:
Mithilfe einer mit der Zuführungsleitung 15 verbundenen Fluidquelle (nicht dargestellt) wird die Zuführungsleitung 15 mit Fluid, insbesondere Gas, beispielsweise N₂O oder CO₂, beaufschlagt, wobei das Fluid an dem distalen Arbeitsende 43 des kryochirurgischen Instruments 10 von einem rohrförmigen Stufenabschnitt 30n-2, 30n-1, 30n durch den angrenzenden Übergangsabschnitt 32n-2, 32n-1 in Richtung Mündung 22 und Expansionskammer 18 in den darauf folgenden rohrförmigen Stufenabschnitt 30n-2, 30n-1, 30n strömt. Auf Grund der trichterartigen Abnahme des Innenquerschnitts 33 und damit des Strömungsquerschnitts der Zuführungsleitung 15 in den Übergangsabschnitten 32n-2, 32n-1 in Richtung Expansionskammer 18 wird das Fluid in den Übergangsabschnitten 32n-2, 32n-1 beschleunigt. Durch die in den Übergangsabschnitten 32n-2, 32n-1 nicht sprunghafte, sondern sich über eine gewisse Länge hinweg erstreckende, vorzugsweise kontinuierliche oder schrittweise Reduktion des Strömungsquerschnitts 33 von Stufe zu Stufe werden Verwirbelungen und Druckschwankungen aufgrund der Beschleunigung in jedem Übergangsabschnitt 32n-2, 32n-1 weitgehend vermieden. Die Stufenabschnitte 30n-2, 30n-1, 30n weisen vorzugsweise jeweils eine Länge auf, so dass dennoch auftretende Verwirbelungen und/oder Druckschwankungen in dem auf einen Übergangsabschnitt 32n-2, 32n-1 folgenden Stufenabschnitt 30n-2, 30n-1, 30n weitgehend oder vollständig abklingen. Das Gas tritt von dem (n-1)-ten Stufenabschnitt durch den (n-1)-ten Übergangsabschnitt in den Kapillarrohrabschnitt 21 (n-ter Stufenabschnitt) ein. Etwaige Druckschwankungen in dem Gas aufgrund des Übergangs von dem (n-1)-ten Stufenabschnitt auf den Kapillarrohrabschnitt 21 klingen auf Grund der Ausbildung des Kapillarrohrabschnitts 21 vorzugsweise vollständig ab. Es ergibt sich in dem Kapillarrohrabschnitt 21 eine laminare Strömung in Strömungsrichtung 34 zu der Mündung 22 mit entsprechendem Geschwindigkeitsprofil, das sich auf Grund des Abklingens der Druckschwankungen in dem Kapillarrohrabschnitt 21 in dem distalen Endabschnitt des Kapillarrohrabschnitts 21, der an die Mündungsöffnung 22 angrenzt, in Strömungsrichtung 34 vorzugsweise nicht mehr ändert (ungestörtes Strömungsprofil). Der Kapillarrohrabschnitt 21 bildet die Drossel für das Gas für die Ausbildung des Joule-Thomson-Effekts. Auf eine Drosselblende 16, wie in dem Stand der Technik gemäß Fig. 1, die zu einer starken Aufweitung des Fluidstrahls beim Verlassen der Zuführungsleitung 15 in die Expansionskammer 18 und damit zu einer starken Wechselwirkung mit dem rückströmenden Gas führt, kann daher, wie in Figur 2a dargestellt, verzichtet werden. Der Gasstrom strömt aus dem Kapillarrohrabschnitt 21 in die Expansionskammer 18 und aufgrund der Beschleunigung in dem Übergangsabschnitten 32n-2, 32n-1 und der Druckschwankungsfreiheit vor dem Austritt aus der Mündung 22 weit in die Expansionskammer 18 in Richtung zu der gegenüberliegenden Wandfläche 26 des Instrumentenkopfes 12. Dabei strömt das Gas weitgehend ungehindert von dem rückströmenden Gas aus der Mündung 22 aus. Das aus der Mündung 22 strömende und sich in der Expansionskammer 18 entspannende Gas erfährt eine Temperaturverminderung auf Grund des Joule-Thomson-Effekts und kühlt den Kopf 12 und die Anhaftungsfläche 14 derart ab, dass eine Gewebeprobe an der Anhaftungsfläche 14 festgefroren werden kann. Die Gewebeprobe kann daraufhin durch Zug an dem Instrument 10 vom restlichen Gewebe abgetrennt und entnommen werden.

Die Rückströmung des abgekühlten Gases wird entsprechend nicht durch das ausströmende Gas behindert. Das expandierte Gas aus der Expansionskammer strömt vielmehr vorzugsweise parallel zu dem die Zuführungsleitung 21 durch die Mündungsöffnung 22 in die Expansionskammer 18 verlassenden Fluid mit entgegengesetztem Strömungsrichtungssinn aus der Expansionskammer 18 in die Rückführungsleitung 19. Diese großräumige Rückströmung wird in Figur 3, die einen Ausschnitt des Instruments 10 an dessen distalen Ende 13a zeigt, durch die Pfeile veranschaulicht. Das durch die Rückführungsleitung 19 rückströmende Gas streift die äußere Wandfläche des Kapillarrohrabschnitts 21 der Zuführungsleitung 15 und entzieht dem durch den Kapillarleitungsabschnitt 21 strömenden Gas Wärme. Dies wird dadurch unterstützt, dass die Wand 44 des Kapillarrohrabschnitts 21 vorzugsweise so dünn ist, wie die Wand des Stufenabschnitts 30n-1, der an den Übergangsabschnitt 32n-1 auf den Kapillarrohrabschnitt 21 angrenzt, oder sogar dünner.

Das rückströmende Gas kann beispielsweise durch seitliche Öffnungen (nicht gezeigt) in dem Schaft 11 entweichen.

Figur 4 zeigt ausschnittsweise eine abgewandelte beispielhafte Ausführungsform des erfindungsgemäßen Instruments 10. Dargestellt ist ein Endabschnitt 13 des Instruments 10.

Die Zuführungsleitung 15 und die Rückführungsleitung 19 sind in dem Schaft 11 des Instruments 10 nebeneinander ausgebildet. Der Kapillarrohrabschnitt 21 der Zuführungsleitung 15 ist in den in dem Schaft 11 angeordneten Abschnitt der Zuführungsleitung 15 gesteckt. Der Kapillarrohrabschnitt 21 reicht bis in die Kappe 24 des Instruments 10, die die Expansionskammer 18 umgrenzt.

Die Zuführungsleitung 15 weist zumindest drei Stufenabschnitte 30n-2, 30n-1, 30n auf, wobei der letzte Stufenabschnitt 30n von dem Kapillarohrabschnitt 21 gebildet ist. Zumindest in dem Übergangsabschnitt 32n-2 auf den vorletzten Stufenabschnitt 30n-1 nimmt der Innenquerschnitt der Zuführungsleitung 15 in Richtung zu der Mündung 22 in die Expansionskammer 18 trichterförmig ab.

Der Strömungsquerschnitt der an die Expansionskammer 18 angeschlossenen Rückführungsleitung 19 in dem Schaft 11 nimmt in Übergangabschnitten 19m-2, 19m-1 der Rückführungsleitung 19 trichterförmig zu. Zwischen den Übergangsabschnitten 19m-2, 19m-1 der Rückführungsleitung 19 ist der Strömungsquerschnitt in der Rückführungsleitung 19 vorzugsweise weitgehend konstant. Die Anzahl Übergangsabschnitte 19m-2, 19m-1 der Rückführungsleitung 19 kann der Anzahl Übergangsabschnitte 32n-3, 32n-2, 32n-1 in der Zuführungsleitung 15 entsprechen.

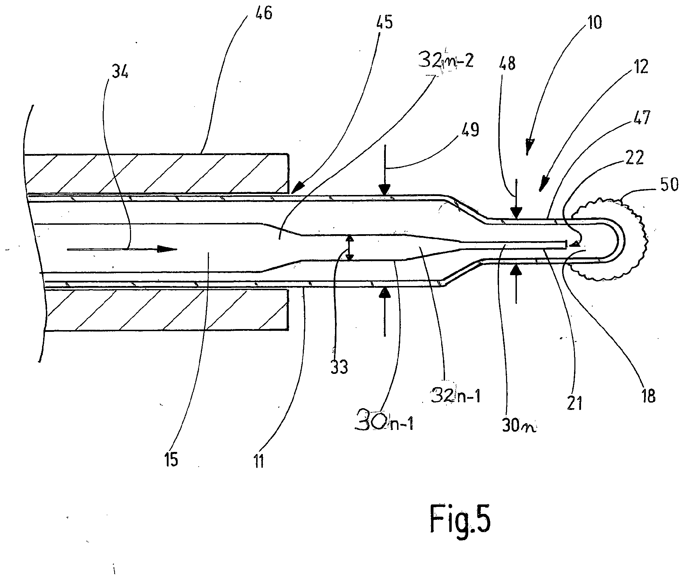

Figur 5 zeigt ein erfindungsgemäßes kryochirurgisches Instrument 10, dessen Schaft 11 in einem Arbeitskanal 45 eines Endoskops 46 längsverschieblich bewegbar geführt ist. An dem distalen Ende des Schaftes 11 des Instruments 10 ist der Kopf 12 des Instruments 10 mit einem schlanken distalen Endabschnitt 47 angeordnet, wobei der Außendurchmesser 48 des Endabschnitts 47 gegenüber dem Außendurchmesser 49 des an den Kopf 12 angrenzenden Schaftabschnitts reduziert ist. Der Kapillarrohrabschnitt 21 ragt in den schmalen Endabschnitt 47, in die Expansionskammer 18 hinein, die durch den Endabschnitt 47 begrenzt wird. In dem Ausführungsbeispiel wird das Fluid in zumindest zwei aufeinander folgenden Übergangsabschnitten 32n-2, 32n-1 der Zuführungsleitung 15 mit jeweils trichterförmiger Verjüngung des Innenquerschnitts 33 in Strömungsrichtung 34 zu der Mündung 22 in die Expansionskammer 18 beschleunigt, wobei sich an jeden Übergangsabschnitt 32n-2, 32n-1 ein rohrförmiger Stufenabschnitt 30n-1, 30n anschließt. Bei dem distal letzten Stufenabschnitt 30n handelt es sich um den Kapillarrohrabschnitt 21. Aufgrund der gleichmäßigen Beschleunigung in den Übergangsabschnitten 32n-2, 32n-1 und aufgrund des Abklingens von Druckschwankungen in dem Kapillarrohrabschnitt 21, so dass das Strömungsprofil des in Richtung zu der Expansionskammer 18 durch die Zuführungsleitung 15 strömenden Fluids am Ende des Kapillarrohrabschnitts 21 in Strömungsrichtung 34 vorzugsweise konstant ist, sich also in Strömungsrichtung 34 nicht mehr verändert, strömt das Fluid nach dem Austritt aus der Mündung 22 weit in die Expansionskammer 18. Aufgrund dessen ist eine geeignete Rückführung des expandierten Gases aus der Expansionskammer 18 ohne Behinderung durch das aus der Mündung 22 in die Expansionskammer 18 ausströmende Gas auch bei den auf Grund des schlanken Endabschnitts 47 des Instrumentenkopfes 12 beengten Platzverhältnissen möglich. Mit dem Instrumentenkopf 12 kann nun eine Gewebeprobe 50 gewonnen werden, deren Durchmesser kleiner ist, als der Durchmesser des Arbeitskanals 45 des Endoskops 46. Damit kann der Kopf 12 des Instruments 10 mit der Gewebeprobe 50 nach der Gewinnung der Gewebeprobe 50 in den Arbeitskanal 45 des Endoskops 46 zurückgezogen werden, so dass die Gewebeprobe 50 in dem Arbeitskanal 45 des Endoskops 46 geschützt aus dem Körper des Patienten geborgen werden kann.

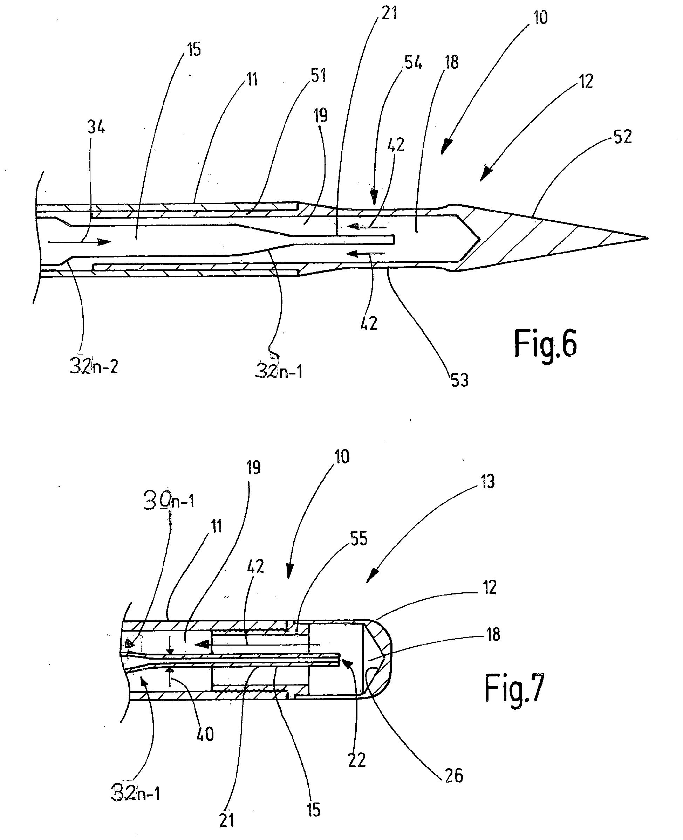

Figur 6 zeigt einen Ausschnitt eines erfindungsgemäßen Instruments 10 mit einem Kopf 12, der mit einem rohrförmigen Befestigungsabschnitt 51 an dem Schaft 11 des Instruments 10 befestigt ist. Der Kopf 12 weist einen spitzen Endabschnitt 52 auf und zwischen Endabschnitt 52 und Befestigungsabschnitt 51 ist ein rohrförmiger Anhaftungsabschnitt 53 angeordnet. Der Kopf 12 weist eine Taille 54 an dem Anhaftungsabschnitt 53 auf. Insbesondere ist der Außendurchmesser des Anhaftungsabschnitts 53 gegenüber dem Außendurchmesser des spitzen Endabschnitts 52 reduziert. Die Wand des Anhaftungsabschnitts 53 weist vorzugsweise eine gegenüber der Wand des Befestigungsabschnitts 51 reduzierte Dicke auf. Der Anhaftungsabschnitt 53 begrenzt die Expansionskammer 18, die bis in den spitzen Endabschnitt 52 reichen kann. Der Kapillarleitungsabschnitt 21 der Zuführungsleitung 15 reicht bis in den Anhaftungsabschnitt 53. Der spitze Endabschnitt 52 erleichtert die Punktion des Gewebes für die Probenentnahme. Zur Entnahme der Probe wird die Zuführungsleitung 15 des Instruments 10 mit Fluid beaufschlagt, wobei das Fluid durch die Zuführungsleitung 15 in Strömungsrichtung zu der Expansionskammer 18 strömt und in der Expansionskammer 18 expandiert und den Kopf 12 abkühlt. Die Gefrierwirkung auf das Gewebe kann dabei insbesondere von dem Anhaftungsabschnitt 53 ausgehen. Die Entnahme der Probe ist vereinfacht, da durch den reduzierten Außendurchmesser des Anhaftungsabschnitts 53 im Vergleich zu dem Außendurchmesser des spitzen Endabschnitts 52 ein Formschluss zwischen dem Kopf 12 und dem festgefrorenen Gewebe gebildet wird.

Figur 7 zeigt einen Ausschnitt des distalen Endes 13 einer beispielhaften Ausführungsform des erfindungsgemäßen Instruments 10 mit einem Kopf 12, der mittels eines Kopfaufnahmeteils 55 an dem Schaft 11 befestigt ist. Das Kopfaufnahmeteil 55 des Instruments 10 erstreckt sich innerhalb des von dem Schaft 11 und dem Kopf 12 umgrenzten Lumens. Der Außendurchmesser des Kapillarleitungsabschnitts 21 ist kleiner als der Außendurchmesser des an den Übergangsabschnitt 32n-1 auf den Kapillarleitungsabschnitt 21 angrenzenden Stufenabschnitts 30n-1. Auf Grund der beispielsgemäß kontinuierlichen Verjüngung des Innenquerschnitts der Zuführungsleitung 15 in dem Übergangsabschnitt 32n-1 auf den Kapillarleitungsabschnitt 21 ist eine Beeinträchtigung des Rückstroms des expandierten Gases durch das aus der Zuführungsleitung 15 ausströmende Fluid weitgehend vermieden. Zudem kann der Strömungswiderstand der Rückführungsleitung 19 durch die kontinuierliche Zunahme des Außenquerschnitts 40 der Zuführungsleitung 15 an dem Übergangsabschnitt 32n-1 (in Strömungsrichtung des von der Expansionskammer 18 wegströmenden Gases) gegenüber einem Instrument mit sprunghafter Zunahme verbessert sein. Durch die Gestaltung der Zuführungsleitung 15 wird demnach trotz der Reduzierung des freien Volumens durch das Kopfaufnahmeteil 55 eine geeignete Rückführung des expandierten Gases ermöglicht. Die der Mündung 22 des Kapillarrohrabschnitts 21 gegenüberliegende, die Expansionskammer 18 begrenzende Wandfläche 26 ist in diesem Ausführungsbeispiel wie auch in dem Ausführungsbeispiel gemäß Figur 6 eine Konusmantelfläche.

Es wird ein kryochirurgisches Instrument 10 angegeben, das eine Zuführungsleitung 15 zum Zuführen von Fluid in eine Expansionskammer 18 des Instruments 10 aufweist. Die Zuführungsleitung 15 weist einen Kapillarleitungsabschnitt 21 auf, der in die Expansionskammer 18 mündet und der eine Drossel für das Fluid zur Ausbildung des Joule-Thompson-Effekts beim Expandieren des Fluids in der Expansionskammer 18 bildet. Der Strömungsquerschnitt der Zuführungsleitung 15 nimmt in zumindest einem Übergangsabschnitt 32n-2, 32n-1, vorzugsweise in zwei oder mehr Übergangsabschnitten 32n-2, 32n-1, der Zuführungsleitung 15 trichterförmig in Strömungsrichtung 34 zu der Expansionskammer 18 ab. Auf jeden Übergangsabschnitt 32n-2, 32n-1 folgt in Strömungsrichtung 34 an den Übergangsabschnitt 32n-2, 32n-1 angrenzend vorzugsweise ein Stufenabschnitt 30n-1, 30n der Zuführungsleitung 15, in dem der Strömungsquerschnitt vorzugsweise weitgehend konstant ist. Der letzte Stufenabschnitt 30n-1, 30n wird vorzugsweise von dem Kapillarleitungsabschnitt 21 gebildet. In den Stufenabschnitten 30n-1, 30n können Druckschwankungen in dem Fluid abklingen. Auf Grund der Beschleunigung des Fluids in den Übergangsabschnitten 32n-2, 32n-1 sowie des Abklingens von Druckschwankungen in dem Kapillarrohrabschnitt 21 und gegebenenfalls in den weiteren Stufenabschnitten 30n-1, 30n-2 wird der Expansionsbereich in der Expansionskammer 18 vergrößert, ohne den Rückstrom des expandierten Gases aus der Expansionskammer 18 zu behindern.

Durch die Verwendung des Kapillarrohrabschnitts 21 sowie des oder der trichterförmigen Übergangsabschnitte 30n-2, 30n-1 wird die freie Weglänge des Fluidstrahls ohne Aufweitung des Fluidstrahls bei dem erfindungsgemäßen Instrument 10 im Vergleich zu einem kryochirurgischen Instrument mit einer Drosselblende am Ende der Zuführungsleitung 15 stark vergrößert, so dass die Wechselwirkung zwischen dem von der Mündungsöffnung 22 weg in die Expansionskammer 18 strömenden Fluid und dem aus der Expansionskammer 18 rückströmenden Gas stark vermindert werden kann. Vorzugsweise klingen Druckschwankungen und/oder Verwirbelungen des durch die Zuführungsleitung 15 in Richtung zu der Mündung 22 strömenden Fluids bei einer Ausführungsform des erfindungsgemäßen Instruments 10 in dem Kapillarrohrabschnitt 21 soweit ab, dass diese die freie Weglänge des Fluidstrahls ohne Aufweitung in der Expansionskammer 18 nicht mehr bestimmen. Die freie Weglänge des Fluidstrahls ohne Aufweitung wird von der Mündungsöffnung 22 in Strömungsrichtung 34 des Fluids bis zu der Stelle in der Expansionskammer 18 gemessen, an der der Fluidstrahldurchmesser einen Betrag überschreitet, der gleich dem Betrag des Außendurchmessers des Kapillarleitungsabschnitts 21 an der Mündungsöffnung 22 ist, oder die freie Weglänge des Fluidstrahls ohne Aufweitung wird von der Mündungsöffnung 22 in Strömungsrichtung 34 des Fluids bis zu der Stelle in der Expansionskammer 18 gemessen, auf Höhe (in Strömungsrichtung 34) der eine Wechselwirkung des von der Mündungsöffnung 22 weg in die Expansionskammer 18 strömenden Fluidstrahls mit dem zu der Rückführungsleitung 19 rückströmenden Gas einsetzt.

**Bezugszeichenliste:**

| | |
|---|---|
| 10 | Instrument |
| 11 | Schaft |
| 12 | Kopf |
| 13 | Distaler Endabschnitt des Instruments |
| 13a | Distales Ende des Instruments |
| 14 | Anhaftungsfläche |
| 15 | Zuführungsleitung |
| 16 | Drosselblende |
| 17 | Öffnung |
| 18 | Expansionskammer |
| 19 | Rückführungsleitung |
| 19m-2, 19m-1 | Übergangsabschnitte der Rückführungsleitung |
| 20 | Distales Ende der Zuführungsleitung |
| 21 | Kapillarleitungsabschnitt/Kapillarrohrabschnitt |
| 22 | Mündung |
| 23 | Stirnseite |
| 24 | Kappe |
| 25 | Abstand |
| 26 | Wandfläche |
| 27 | Lumen |
| 28 | Durchmesser |
| 29 | Länge |
| 30n-2, 30n-1, 30n | Stufenabschnitt |
| 32n-2, 32n-1 | Übergangsabschnitt |
| 33 | Innenquerschnittsfläche/Strömungsquerschnittsfläche |
| 34 | Strömungsrichtung zur Expansionskammer |
| 35 | Innere Wandfläche |
| 36 | Übergangsbereich |
| 37 | Verjüngungswinkel |
| 38a | Wand des Schaftes |
| 38b | Wand des Kopfes |
| 39 | Wand der Zuführungsleitung |
| 40 | Außenquerschnitt |
| 41 | Strömungsquerschnitt der Rückführungsleitung |
| 42 | Strömungsrichtung von der Expansionskammer weg |
| 43 | distales Arbeitsende |
| 44 | Wand des Kapillarrohrabschnitts |
| 45 | Arbeitskanal |
| 46 | Endoskop |
| 47 | Endabschnitt |
| 48 | Außendurchmesser Endabschnitt |
| 49 | Außendurchmesser Schaft |
| 50 | Gewebeprobe |
| 51 | Befestigungsabschnitt |
| 52 | Endabschnitt |
| 53 | Anhaftungsabschnitt |
| 54 | Taille |
| 55 | Kopfaufnahmeteil |
| | |
| An-2, An-1, An | Strömungsquerschnittsflächeninhalt der Rückführungsleitung |
| Bn-2, Bn-1, Bn | Strömungsquerschnittsflächeninhalt der Zuführungsleitung |
| S₁-S₁, S₂-S₂, S₃-S₃ | Schnittebenen |

## Patentansprüche

1. Kryochirurgisches Instrument (10)
mit einer Zuführungsleitung (15) zum Zuführen von Fluid, insbesondere Gas, in eine Expansionskammer (18), mit einer an die Expansionskammer (18) angeschlossenen Rückführungseinrichtung (19) zum Rückführen von Gas aus der Expansionskammer (18),
wobei die Zuführungsleitung (15) wenigstens einen ersten Abschnitt (30n-2, 30n-1) und einen zweiten Abschnitt (30n-1, 30n) mit unterschiedlich großen Innenquerschnitten (33) aufweist,
wobei sich der Innenquerschnitt (33) der Zuführungsleitung (15) in einem Übergangsabschnitt (32n-2, 32n-1) von dem ersten Abschnitt (30n-2, 30n-1) auf den zweiten Abschnitt (30n-1, 30n) in Strömungsrichtung (34) des Fluids zu der Expansionskammer (18) trichterförmig verjüngt,
wobei der Außenquerschnitt der Zuführungsleitung an dem trichterförmigen Übergangsabschnitt in Strömungsrichtung des von der Expansionskammer wegströmenden Gases nach der Expansion gesehen nicht sprunghaft zunimmt, sondern kontinuierlich und/oder schrittweise,
**dadurch gekennzeichnet, dass** die Zuführungsleitung (15) einen Kapillarleitungsabschnitt (21) aufweist, der in die Expansionskammer (18) mündet und der eine Drossel für das Fluid für die Ausbildung des Joule-Thomson-Effekts an dem Fluid bei der Expansion des Fluids in die Expansionskammer bildet.

2. Kryochirurgisches Instrument (10) nach Anspruch 1, wobei sich der Strömungsweg in der Zuführungsleitung (15) in dem Übergangabschnitt (32n-1) auf den Kapillarleitungsabschnitt (21, 30n) trichterförmig verjüngt.

3. Kryochirurgisches Instrument (10) nach wenigstens einem der vorhergehenden Ansprüche, wobei die Zuführungsleitung (15) wenigstens zwei Übergangsabschnitte (32n-2, 32n-1) aufweist, in denen sich der Strömungsweg der Zuführungsleitung (15) in Strömungsrichtung (34) trichterförmig verjüngt.

4. Kryochirurgisches Instrument (10) nach wenigstens einem der vorhergehenden Ansprüche, wobei der erste Abschnitt (30n-2, 30n-1, 30n) und der zweite Abschnitt (30n-2, 30n-1, 30n) Stufenabschnitte (30n-2, 30n-1, 30n) einer Reihe von zwei oder mehr als zwei Stufenabschnitten (30n-2, 30n-1, 30n) der Zuführungsleitung (15) sind, wobei zwischen zwei Stufenabschnitten (30n-2, 30n-1, 30n) je ein Übergangsabschnitt (32n-2, 32n-1) angeordnet ist, an den die beiden Stufenabschnitte (30n-2, 30n-1, 30n) angrenzen, wobei die Flächeninhalte der Innenquerschnittsflächen jedes Stufenabschnitts (30n-2, 30n-1, 30n) einer Innenquerschnittstufe angehören, wobei die Flächeninhalte der Innenquerschnittsflächen einer Innenquerschnittsstufe eines Stufenabschnitts (30n-2, 30n-1, 30n) größer sind als die Flächeninhalte der Innenquerschnittsflächen der Innenquerschnittsstufe des in Richtung (34) zu der Mündung (22) des Kapillarleitungsabschnitts (21) in Strömungsrichtung an denselben Übergangsabschnitt (32n-2, 32n-1) angrenzenden Stufenabschnitts (30n-2, 30n-1, 30n).

5. Kryochirurgisches Instrument (10) nach wenigstens einem der vorhergehenden Ansprüche, wobei das Instrument (10) derart ausgebildet ist, dass der Strömungsquerschnitt (33) für das Fluid beim Übergang von der Mündung (22) des Kapillarleitungsabschnitts (21) in die Expansionskammer (18) sprunghaft ansteigt.

6. Kryochirurgisches Instrument (10) nach wenigstens einem der vorhergehenden Ansprüche, wobei die Zuführungsleitung (15) in einer Rückführungsleitung (19) der Rückführungseinrichtung (19) angeordnet ist und/oder wobei die Rückführungsleitung (19) neben der Zuführungsleitung (15) angeordnet ist, wobei das Verhältnis des Strömungsquerschnitts (41) der Rückführungsleitung (19) neben dem Kapillarleitungsabschnitt (21) oder um den Kapillarleitungsabschnitt (21) herum zu dem Innenquerschnitt (33) des Kapillarleitungsabschnitts (21) größer oder gleich 5 ist.

7. Kryochirurgisches Instrument (10) nach wenigstens einem der vorhergehenden Ansprüche, wobei der Außenquerschnitt (40) der Zuführungsleitung (15) an dem trichterförmigen Übergangsabschnitt (32n-2, 32n-1) oder den trichterförmigen Übergangsabschnitten (32n-2, 32n-1) kontinuierlich in Richtung (34) zu der Mündung (22) des Kapillarleitungsabschnitts (21) abnimmt.

8. Kryochirurgisches Instrument (10) nach wenigstens einem der vorhergehenden Ansprüche, wobei der Abschnitt der Zuführungsleitung (15), der den Kapillarleitungsabschnitt (21) und die Übergangsabschnitten (32n-2, 32n-1) enthält, nahtlos einstückig ausgebildet ist.

9. Kryochirurgisches Instrument (10) nach wenigstens einem der vorhergehenden Ansprüche, wobei der Abschnitt der Zuführungsleitung (15) mit dem Kapillarleitungsabschnitt (21) und den trichterförmigen Übergangsabschnitten (32n-2, 32n-1) mit einem Rundknetverfahren hergestellt ist.

10. Kryochirurgisches Instrument (10) nach wenigstens einem der vorhergehenden Ansprüche, wobei die Wandstärke des Kapillarleitungsabschnitts (21) gleich oder geringer als die Wandstärke des Zuführungsleitungsabschnitts angrenzend an den Übergangsabschnitt (32n-2, 32n-1) auf den Kapillarleitungsabschnitt (21) ist.

11. Kryochirurgisches Instrument (10) nach wenigstens einem der vorhergehenden Ansprüche, wobei das Verhältnis des Innendurchmessers (28) der Kapillarleitungsabschnitts (21) zu der Länge (29) des Kapillarleitungsabschnitts (21) zwischen minimal 0,004 bis maximal 0,2 beträgt.

12. Kryochirurgisches Instrument (10) nach wenigstens einem der vorhergehenden Ansprüche, wobei der Verjüngungswinkel (37), mit dem sich der Innenquerschnitt (33) in dem Übergangsabschnitt (32n-2, 32n-1) trichterartig verjüngt, zwischen minimal 15° und maximal 40° beträgt.

13. Kryochirurgisches Instrument (10) nach wenigstens einem der vorhergehenden Ansprüche, wobei die Mündungsöffnung (22) des Kapillarleitungsabschnitts (21) an der Stirnseite (23) des Kapillarleitungsabschnitts (21) angeordnet ist.

14. Kryochirurgisches Instrument (10) nach wenigstens einem der vorhergehenden Ansprüche, wobei der Abstand (25) zwischen der Mündungsöffnung (22) und der gegenüberliegenden Wandfläche (26) der Expansionskammer (18) zwischen minimal 0,5 Millimeter und maximal 5 Millimeter beträgt.

## Claims

1. Cryosurgical instrument (10)
having a feed line (15) for supplying fluid, in particular gas, into an expansion chamber (18),
having a return system (19) connected to the expansion chamber (18) for returning gas out of the expansion chamber (18),
wherein the feed line (15) has at least one first section (30n-2, 30n-1) and one second section (30n-1, 30n) with different-size inside cross-sections (33),
wherein the inside cross-sectional area (33) of the feed line (15) tapers in the form of a funnel in a transition section (32n-2, 32n-1) from the first section (30n-2, 30n-1) to the second section (30n-1, 30n) in the direction of flow (34) of the fluid toward the expansion chamber (18),
wherein viewed in the direction of flow of the gas flowing away from the expansion chamber following expansion, the outside cross-section of the feed line in the funnel-shaped transition section does not increase abruptly but continuously and/or step-by-step,
**characterized in that** the feed line (15) has a capillary line section (21) that terminates in the expansion chamber (18) and that has an aperture for the fluid to form the Joule-Thomson effect on the fluid during the expansion of the fluid in the expansion chamber (18).

2. Cryosurgical instrument (10) according to Claim 1, wherein the path of flow in the feed line (15) tapers in the form of a funnel in the transition section (32n-1) toward the capillary line section (21, 30n).

3. Cryosurgical instrument (10) according to at least one of the previous claims, wherein the feed line (15) has at least two transition sections (32n-2, 32n-1), in which the flow path of the feed line (15) tapers in the form of a funnel in the direction of flow (34).

4. Cryosurgical instrument (10) according to at least one of the previous claims, wherein the first section (30n-2, 30n-1, 30n) and the second section (30n-2, 30n-1, 30n) are step sections (30n-2, 30n-1, 30n) of a series of two or more than two step sections (30n-2, 30n-1, 30n) of the feed line (15), wherein - between two step sections (30n-2, 30n-1, 30n) - respectively one transition section (32n-2, 32n-1) is arranged, said transition section being adjacent to the two step sections (30n-2, 30n-1, 30n), wherein the areas of the inside cross-sections (33) of each step section (30n-2, 30n-1, 30n) belong to an inside cross-section step, wherein the areas of the inside cross-sections of an inside cross-section step of a step section (30n-2, 30n-1, 30n) are greater than the areas of the inside cross-sections of the inside cross-section step of the step section (30n-2, 30n-1, 30n) adjoining the same transition section (32n-2, 32n-1) in the direction (34) toward the mouth (22) of the capillary line section (21) in the direction of flow.

5. Cryosurgical instrument (10) according to at least one of the previous claims, wherein the instrument (10) is configured in such a manner that the flow cross-section (33) for the fluid increases abruptly during the transition from the mouth (22) of the capillary line section (21) into the expansion chamber (18) .

6. Cryosurgical instrument (10) according to at least one of the previous claims, wherein feed line (15) is arranged in a return line (19) of the return system (19) and/or wherein the return line (19) is arranged next to the return line (15), wherein the ratio of the flow cross-section (41) of the return line (19) next to the capillary line section (21) or around the capillary line section (21) with respect to the inside cross-section (33) of the capillary line section (21) is greater than or equal to 5.

7. Cryosurgical instrument (10) according to at least one of the previous claims, wherein the outside cross-section (40) of the feed line (15) decreases continuously in the funnel-shaped transition section (32n-2, 32n-1) or the funnel-shaped transition sections (32n-2, 32n-1) in the direction (34) toward the mouth (22) of the capillary line section (21).

8. Cryosurgical instrument (10) according to at least one of the previous claims, wherein the section of the feed line (15) comprising the capillary line section (21) and the transition sections (32n-2, 32n-1) are formed in one piece without seam.

9. Cryosurgical instrument (10) according to at least one of the previous claims, wherein the section of the feed line (15) having the capillary line section (21) and the funnel-shaped transition sections (32n-2, 32n-1) is produced with the use of a rotary swaging process.

10. Cryosurgical instrument (10) according to at least one of the previous claims, wherein the wall thickness of the capillary line section (21) is equal to or greater than the wall thickness of the feed line section adjacent to the transition section (32n-2, 32n-1) toward the capillary line section (21).

11. Cryosurgical instrument (10) according to at least one of the previous claims, wherein the ratio of the inside diameter (28) of the capillary line section (21) with respect to the length (29) of the capillary line section (21) is between a minimum of 0.004 up to a maximum of 0.2.

12. Cryosurgical instrument (10) according to at least one of the previous claims, wherein the tapering angle (37) at which the inside cross-section (33) tapers funnel-like in the transition section (32n-2, 32n-1) is between 15° at minimum and 40° at maximum.

13. Cryosurgical instrument (10) according to at least one of the previous claims, wherein the mouth opening (22) of the capillary line section (21) is provided on the front side (23) of the capillary line section (21).

14. Cryosurgical instrument (10) according to at least one of the previous claims, wherein the distance (25) between the mouth opening (22) and the opposite wall surface (26) of the expansion chamber (18) is between 0.5 millimeters at minimum and 5 millimeters at maximum.

## Revendications

1. Instrument cryochirurgical (10),
comprenant une conduite d'alimentation (15) destinée à amener du fluide, en particulier du gaz, dans une chambre d'expansion (18), comprenant un dispositif de retour (19) raccordé à la chambre d'expansion (18) et destiné à ramener du gaz depuis la chambre d'expansion (18),
dans lequel la conduite d'alimentation (15) présente au moins une première portion (30n-2, 30n-1) et une deuxième portion (30n-1, 30n) ayant des sections intérieures (33) différentes,
dans lequel la section intérieure (33) de la conduite d'alimentation (15) diminue en forme d'entonnoir dans une portion de transition (32n-2, 32n-1) de la première portion (30n-2, 30n-1) à la deuxième portion (30n-1, 30n), dans le sens d'écoulement (34) du fluide vers la chambre d'expansion (18),
dans lequel la section extérieure de la conduite d'alimentation n'augmente pas brusquement à l'endroit de la portion de transition en forme d'entonnoir, dans le sens d'écoulement du gaz s'écoulant depuis la chambre d'expansion, vu après l'expansion, mais augmente de manière continue et/ou graduellement,
**caractérisé en ce que** la conduite d'alimentation (15) présente une portion de conduite capillaire (21) qui débouche dans la chambre d'expansion (18) et qui constitue un élément d'étranglement pour le fluide, en vue de la formation de l'effet de Joule-Thomson sur le fluide, lors de l'expansion du fluide dans la chambre d'expansion.

2. Instrument cryochirurgical (10) selon la revendication 1, dans lequel la voie d'écoulement dans la conduite d'alimentation (15) se rétrécit en forme d'entonnoir dans la portion de transition (32n-1) vers la portion de conduite capillaire (21, 30n).

3. Instrument cryochirurgical (10) selon au moins une des revendications précédentes, dans lequel la conduite d'alimentation (15) présente au moins deux portions de transition (32n-2, 32n-1) dans lesquelles la voie d'écoulement de la conduite d'alimentation (15) se rétrécit en forme d'entonnoir dans le sens d'écoulement (34).

4. Instrument cryochirurgical (10) selon au moins une des revendications précédentes, dans lequel la première portion (30n-2, 30n-1, 30n) et la deuxième portion (30n-2, 30n-1, 30n) sont des portions en gradins (30n-2, 30n-1, 30n) d'une série de deux ou plus de deux portions en gradins (30n-2, 30n-1, 30n) de la conduite d'alimentation (15), sachant qu'il est prévu entre deux portions en gradins (30n-2, 30n-1, 30n) chaque fois une portion de transition (32n-2, 32n-1) que jouxtent les deux portions en gradins (30n-2, 30n-1, 30n), sachant que les aires des surfaces de section intérieure de chaque portion en gradin (30n-2, 30n-1, 30n) font partie d'un gradin de section intérieure, les aires des surfaces de section intérieure d'un gradin de section intérieure d'une portion en gradin (30n-2, 30n-1, 30n) étant supérieures aux aires des surfaces de section intérieure du gradin de section intérieure de la portion en gradin (30n-2, 30n-1, 30n) qui jouxte la même portion de transition (32n-2, 32n-1) dans le sens d'écoulement en direction de l'embouchure (22) de la portion de conduite capillaire (21).

5. Instrument cryochirurgical (10) selon au moins une des revendications précédentes, dans lequel l'instrument (10) est réalisé de manière telle que la section d'écoulement (33) pour le fluide augmente brusquement lors de la transition depuis l'embouchure (22) de la portion de conduite capillaire (21) vers la chambre d'expansion (18).

6. Instrument cryochirurgical (10) selon au moins une des revendications précédentes, dans lequel la conduite d'alimentation (15) est disposée dans une conduite de retour (19) du dispositif de retour (19), et/ou dans lequel la conduite de retour (19) est disposée à côté de la conduite d'alimentation (15), le rapport de la section d'écoulement (41) de la conduite de retour (19), à côté de la portion de conduite capillaire (21) ou autour de la portion de conduite capillaire (21), à la section intérieure (33) de la portion de conduite capillaire (21) étant supérieur ou égal à 5.

7. Instrument cryochirurgical (10) selon au moins une des revendications précédentes, dans lequel la section extérieure (40) de la conduite d'alimentation (15), à l'endroit de la portion de transition (32n-2, 32n-1) en forme d'entonnoir ou des portions de transition (32n-2, 32n-1) en forme d'entonnoirs, diminue de manière continue en direction (34) de l'embouchure (22) de la portion de conduite capillaire (21).

8. Instrument cryochirurgical (10) selon au moins une des revendications précédentes, dans lequel la portion de la conduite d'alimentation (15) qui comporte la portion de conduite capillaire (21) et les portions de transition (32n-2, 32n-1) est réalisée d'une seule pièce, sans soudure.

9. Instrument cryochirurgical (10) selon au moins une des revendications précédentes, dans lequel la portion de la conduite d'alimentation (15) qui comporte la portion de conduite capillaire (21) et les portions de transition (32n-2, 32n-1) en forme d'entonnoirs est fabriquée par un procédé de rétreint rotatif.

10. Instrument cryochirurgical (10) selon au moins une des revendications précédentes, dans lequel l'épaisseur de paroi de la portion de conduite capillaire (21) est égale ou inférieure à l'épaisseur de paroi de la portion de la conduite d'alimentation qui jouxte la portion de transition (32n-2, 32n-1) vers la portion de conduite capillaire (21).

11. Instrument cryochirurgical (10) selon au moins une des revendications précédentes, dans lequel le rapport du diamètre intérieur (28) de la portion de conduite capillaire (21) à la longueur (29) de la portion de conduite capillaire (21) est compris entre au minimum 0,004 et au maximum 0,2.

12. Instrument cryochirurgical (10) selon au moins une des revendications précédentes, dans lequel l'angle de rétrécissement (37) avec lequel la section intérieure (33) diminue en forme d'entonnoir dans la portion de transition (32n-2, 32n-1) est compris entre au minimum 15° et au maximum 40°.

13. Instrument cryochirurgical (10) selon au moins une des revendications précédentes, dans lequel l'orifice d'embouchure (22) de la portion de conduite capillaire (21) est disposé sur la face frontale (23) de la portion de conduite capillaire (21).

14. Instrument cryochirurgical (10) selon au moins une des revendications précédentes, dans lequel la distance (25) entre l'orifice d'embouchure (22) et la surface de paroi (26), située en vis-à-vis, de la chambre d'expansion (18) est comprise entre au minimum 0,5 millimètres et au maximum 5 millimètres.
